# EUROPEAN PATENT SPECIFICATION

(11) **EP 1 877 432 B1**
(45) Date of publication and mention of the grant of the patent: **07.04.2010**
(21) Application number: 06750874.7
(22) Date of filing: 20.04.2006
(51) Int. Cl.: C07K 14/47, G01N 33/50

(54) **STABILIZATION OF CARDIAC TROPONIN**
STABILISIERUNG VON HERZ-TROPONIN
STABILISATION DE LA TROPONINE CARDIAQUE

(30) Priority: 28.04.2005 US 675542 P
(43) Date of publication of application: 16.01.2008
(73) Proprietor: ABBOTT LABORATORIES, Abbott Park IL 60064-3500 (US)
(72) Inventor: LAIRD, Don, M., Mundelein, Illinois 60060 (US); YOUNG, Charles, E., Bellwood, Illinois 60104 (US)
(74) Representative: Modiano, Micaela Nadia
(86) International application number: PCT/US2006/014958
(87) International publication number: WO 2006/116005

(56) References cited:
- WO-A-96/27661
- WO-A-98/56900
- WO-A-02/064115
- US-A- 5 834 210
- US-A- 5 851 554
- US-A1- 2001 006 683
- US-A1- 2002 193 287
- BODOR G S ET AL: "DEVELOPMENT OF MONOCLONAL ANTIBODIES FOR AN ASSAY OF CARDIAC TROPONIN-I AND PRELIMINARZ RESULTS IN SUSPECTED CASES OF MYOCARDIALINFARCTION" CLINICAL CHEMISTRY, AMERICAN ASSOCIATION FOR CLINICAL CHEMISTRY, WASHINGTON, DC, US, vol. 38, no. 11, 1 November 1992 (1992-11-01), pages 2203-2214, XP000470202 ISSN: 0009-9147

## Description

The invention relates to a stabilized composition of cardiac troponin protein useful as calibrator and control standards in immunoassays. Particularly, the troponin protein is stabilized in an aqueous matrix containing at least one anionic surfactant.

### BACKGROUND OF THE INVENTION

The Troponin complex plays a role in the calcium-dependent regulation of muscle contraction and relaxation. Three distinct proteins, or isoforms of Troponin, comprise the Troponin complex, and can be found in both cardiac and skeletal muscles. These three proteins are designated as Troponin-I, Troponin-C and Troponin-T. During myocardial infarction, cardiac muscle cells die and consequently release their intracellular contents, including the Troponin proteins, into the blood stream.

Myocardial infarction is a leading cause of death in developed countries. The World Health Organization (WHO) developed guidelines for diagnosing myocardial infarction in 1979 (See Circulation, 59:607-609 (1979)). The WHO guidelines recommend that a diagnosis of myocardial infarction be dependent on the occurrence of two of three particular criteria. The three criteria are: (1) chest pain or history of cardiac event; (2) electrocardiogram indication of cardiac event; and (3) elevated levels of the enzyme creatine kinase.

Millions of individuals enter emergency rooms each year complaining of chest pain and have non-diagnostic electrocardiograms thus making difficult a diagnosis of cardiac event. Measurement of circulating levels of creatine kinase has questionable specificity relative to occurrence of myocardial infarction since elevation of this protein in the blood could also result from skeletal muscle damage. Troponin-I and Troponin-T have shown greater specificity due to the presence of a cardiac specific form of these proteins present only in the heart. The greater specificity of Troponin-I and Troponin-T for diagnosis of myocardial infarction has led to the development of several immunoassays for the determination of the levels of these proteins in blood samples of a patient expected to exhibit elevated levels. The literature demonstrating the superior utility of troponin has resulted in an updating of the definition of myocardial infection as of 2000 (which was jointly published in the Journal of the American College of Cardiology, 36:959-969 (2000) and in the European Heart Journal, 21:1502-1513-(2000)). This updated definition places a greater emphasis on biomarkers for diagnosis. As a result, the criteria for an acute, evolving or recent myocardial infarction are the typical rise and gradual fall (troponin) or more rapid rise and fall (CK-MB) of biochemical markers of myocardial necrosis with at least one of the following: (a) ischemic syndromes; (b) development of pathologic Q waves on the ECG, (c) ECG changes indicative o ischemia (ST segment elevation or depression); or (d) coronary artery intervention (e.g. coronary angioplasty).

Quantitative immunoassays require the use of both calibration standards to define a calibration curve and control standards to test the integrity of the calibration curve. One necessary characteristic of the standards used is stability, i.e. demonstrate minimal loss of immunoactivity over a defined period of time (expiration date) under appropriate storage conditions. Stabilization techniques for Troponin-I have included freezing, lyophilization and dissolution in strong reducing agents such as guanidine. These techniques generally require additional time, specialized equipment and special handling procedures required for thawing or reconstitution. Additionally, thawing or reconstitution of the troponin protein often results in unstable material with limited shelf-life.

In US 5834210 it is described a method for the preparation of stable human cardiac Troponin complexes obtained from recombinant modified Troponin-I, recombinant Troponin-C, and recombinant Troponin-T in the presence of an alkaline earth salt or salts and, if desired, a detergent such as sodium dodecyl sulfate.

In WO 96/27661 compositions for stabilizing proteins and fragments of the proteins, such as Troponin and Troponin fragments, are described. The compositions comprise an aqueous solution of a buffer, a stabilizing protein, a chelating agent, a reducing agent and a salt, and can also comprise sodium dodecyl sulfate as a detergent.

Due to the inherent instability of the troponin protein there exists a need for material that is resistant to temperature variations potentially subjected to during transport and storage. There is further a need for a method of preparing such stabilized troponin. The invention provides a stabilized troponin protein and a method of preparing the stabilized troponin.

### SUMMARY OF THE INVENTION

In one embodiment, the invention provides a diagnostic assay standard comprising an aqueous solution of Troponin-1 protein and a matrix comprising at least one anionic surfactant wherein the anionic surfactant in this diagnostic assay standard is sodium polyoxyethylene (1) lauryl sulfate

In yet another further embodiment, the diagnostic assay standard is stable at room temperature for at least six (6) months.

In still yet another further embodiment, the matrix in the diagnostic assay standard further comprises porcine gelatin.

In still another further embodiment, the matrix in the diagnostic assay standard further comprises a compound selected from the group consisting of sodium chloride and sodium phosphate.

In another embodiment, the matrix of the diagnostic assay standard further comprises a pH buffer added to maintain the pH within a range of about 6.8 to about 7.2.

In a further embodiment, the matrix contains sodium polyoxyethylene (1) lauryl sulfate in a concentration of about 0.1 % to about 0.25 %.

In another embodiment, the invention provides a method for stabilizing Troponin in an aqueous solution, the method comprising the steps of:
(a) preparing an aqueous solution of at least one anionic surfactant wherein the anionic surfactant is sodium polyoxyethylene (1) lauryl sulfate;
(b) adding Troponin-I protein to the aqueous solution; and
(c) storing the solution at room temperature.

In yet another embodiment, the invention relates to a diagnostic assay standard comprising an aqueous solution of a complex of a Troponin-I protein and a Troponin-C protein and a matrix comprising at least one anionic surfactant wherein the anionic surfactant in this diagnostic assay standard is sodium polyoxyethylene (1) lauryl sulfate.

In a further embodiment, this diagnostic assay standard is stable at room temperature for at least six (6) months.

In still yet another further embodiment, the matrix in the diagnostic assay standard further comprises porcine gelatin.

In still another further embodiment, the matrix in the diagnostic assay standard further comprises a compound selected from the group consisting of sodium chloride and sodium phosphate.

In another embodiment, the matrix of the diagnostic assay standard further comprises a pH buffer added to maintain the pH within a range of about 6.8 to about 7.2.

In a further embodiment of this diagnostic assay standard, the matrix contains sodium polyoxyethylene (1) lauryl sulfate in a concentration of about 0.1% to about 0.25%.

In another embodiment, the invention provides a method for stabilizing Troponin in an aqueous solution, the method comprising the steps of:
(a) preparing an aqueous solution of at least one anionic surfactant wherein the anionic surfactant is sodium polyoxyethylene (1) lauryl sulfate;
(b) adding Troponin-I protein and Troponin-C protein, either individually or together as a single complex to the aqueous solution; and
(c) storing the solution at room temperature.

### BRIEF DESCRIPTION OF THE DRAWING

Figure 1 is a graph comparing the activity levels of Troponin-I in formulations with and without sodium polyoxyethylene (1) lauryl sulfate added.
Figure 2 is a graph showing lot-to-lot variability of sodium polyoxyethylene (1) lauryl sulfate as STANDAPOL® ES-1 on Troponin-I stability over time at 2-3°C.
Figure 3 is a graph showing lot-to-lot variability of sodium polyoxyethylene (1) lauryl sulfate as STANDAPOL® ES-1 on Troponin-I stability over time at room temperature, 31°C.
Figure 4 is a graph showing lot-to-lot variability sodium polyoxyethylene (1) lauryl sulfate as STANDAPOL® ES-1 on Troponin-I stability over time at 45°C.
Figure 5 is a graph depicting % Troponin-I activity remaining for Low, Medium and High Controls calculated from a calibration curve generated using calibrator standards that were stored frozen and freshly thawed.
Figure 6 is a graph depicting % Troponin-I activity remaining for Low, Medium and High Controls calculated from a calibration curve generated using calibrator standards that were stored at 2-8°C.
Figure 7 is a graph depicting % Troponin-I activity remaining for Low, Medium and High Controls calculated from a calibration curve generated using calibrator standards that were stored at room temperature (31°C).
Figure 8 is a graph depicting % Troponin-I activity remaining for Low, Medium and High Controls that were stored frozen. The concentration of the controls was calculated from a standard curve generated using calibrator standards that were stored frozen and used immediately upon thawing.
Figure 9 is a graph depicting % Troponin-I activity remaining for Low, Medium and High Controls that were stored at 2-8°C. The concentration of the controls was calculated from a standard curve generated using calibrator standards that were frozen and used immediately upon thawing.
Figure 10 is a graph depicting % Troponin-I activity remaining for Low, Medium and High Controls that were stored at worst case room temperature (31°C). The concentration of the controls was calculated from a standard curve generated using calibrator standards that were frozen and used immediately upon thawing.
Figure 11 (A-C) are graphs depicting Troponin-I concentration for Medium Control stored frozen continuous, at 2-8 °C and at worst case room temperature (31°C). The concentration of the controls was calculated from a standard curve generated using calibrator standards that were frozen and used immediately upon thawing.

### DETAILED DESCRIPTION OF THE INVENTION

The present invention is directed to a stabilized Troponin-I protein in a matrix comprising at least one anionic surfactant that is useful as calibration and control standards in immunoassays intended to determine the levels of troponin protein in blood samples of patients suspected of having elevated troponin levels. The present invention also relates to methods of preparing a stabilized troponin protein in a matrix comprising at least one anionic surfactant.

The at least one anionic surfactant used in the matrix and methods of the present invention is sodium polyoxyethylene (1) lauryl sulfate Troponin proteins, particularly cardiac Troponin-I (cTnI), are unstable molecules that rapidly lose immunoactivity in aqueous environments. Immunoactivity stability is an essential characteristic for an analyte that is to be used to prepare calibrators or controls for a diagnostic assay. The inventors of the present invention found that sodium polyoxyethylene (1) lauryl sulfate is capable of imparting stability to cTnI molecules. More specifically, the inventors discovered that the addition of sodium polyoxyethylene (1) lauryl sulfate, to cTnI, imparts thermal stability and robustness to formation of aqueous standard solutions used in calibrators and test assay controls. Robustness refers to the strength of the properties of the troponin. Robustness also may refer to the way in which the troponin has been constructed.

Sodium polyoxyethylene (1) lauryl sulfate provides stability to Troponin-I allowing it to be robust to elevated temperatures, wide ranges in ionic strengths, and pH. Anionic surfactants such as sodium polyoxyethylene (1) lauryl sulfate are commercially available. For example, sodium polyoxyethylene (1) lauryl sulfate is available as STANDAPOL® ES-1 from Cognis Corporation, Hoboken, NJ.

Sodium polyoxyethylene (1) lauryl sulfate can be used as a diluent component for solutions containing troponin and do not require any additional processing. The matrix containing the at least one anionic surfactant and troponin proteins have no special handling requirements by the user and the user may store the matrix at temperatures of between 2-8°C or at room temperatures of up to 31°C for periods of at least six (6) months, one (1) year or more.

Sodium polyoxyethylene (1) lauryl sulfate is believed to be readily soluble in aqueous conditions and can be added and mixed until dissolved. Concentration ranges of the at least one anionic surfactant in the matrix is between about 0.1 % and 0. 25% at a pH in the range of 6.8-7.2 and preferably, 7.0.

Sodium polyoxyethylene (1) lauryl sulfate stabilizes the immunoactivity of cTnI and recombinant Troponin-I/Troponin-C complexes that are formed as a single protein molecule. The recombinant complex can be used in the formulation of calibrators and controls for the assays conducted with, for example, AxSYM Troponin-I *ADV* and ARCHITECT Troponin-I assays. The present invention also contemplates that the matrix containing sodium polyoxyethylene (1) lauryl sulfate can also be used to stabilize the immunoactivity of native or recombinant Troponin-I/Troponin-C complexes that are formed by adding each of the Troponin-I and Troponin-C together to form a complex.

The following examples further illustrate the invention but, of course, should not be construed as in any way limiting its scope. All references, including publications, patent applications, and patents, cited herein are hereby incorporated by reference to the same extent as if each reference were individually and specifically indicated to be incorporated by reference and were set forth in its entirety herein.

### Example 1- Comparison of Troponin-I stabilization at 37 °C and between 2-8°C with and without STANDAPOL® ES-1.

Troponin-I (Code 67911, Lot 63821P101, Abbott Laboratories, Abbott Park, IL) was diluted in matrices of 0.1 % porcine gelatin (Code 35745, Lot 74281P100, Abbott Laboratories, Abbott Park, IL) at pH 4.0 and 0.1% porcine gelatin (Code 35745, Lot 74281P100) containing STANDAPOL® ES-1 (Lot 1 2L019 from Cognis Corporation, Hoboken, NJ) at pH 6.8 to a final concentration of between 40-50 ng/ml (AxSYM Units are ng/ml). Porcine gelatin is added as a source of protein in order to minimize the loss of troponin during manufacture.

Each formulation was stored at both 2-8°C and 37°C as follows:
Sample A - Troponin-I in porcine gelatin at 2-8°C.
Sample B - Troponin-I in porcine gelatin at 37°C
Sample C - Troponin-I in porcine gelatin at 2-8°C with 0.1% STANDAPOL® ES-1.
Sample D - Troponin-I in porcine gelatin at 37°C with 0.1% STANDAPOL® ES-1.

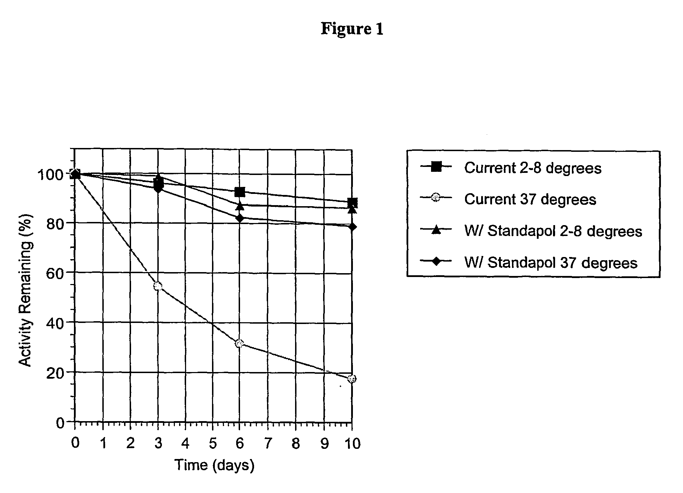

The concentration of Troponin-I in each sample was measured using a standard curve generated on day 0 using AxSYM Troponin-I standard calibration (No. 3C29-01, Abbott Laboratories, Abbott Park, IL) on the AxSYM instrument system (Clinical Chemistry, 45, No. 12, 1999). Over the following ten days, the concentration of Troponin-I was measured and compared to the concentration observed on day 0. The percent activity remaining on each day was calculated by dividing the concentration measured for each sample at the various time points by the concentration measured on day 0. Results are tabulated in Table 1 and depicted in Figure 1.

**Table 1. Stabilization results for Samples A-D.**

| **% Change based on Day 0 Calibration** | | | |
|---|---|---|---|
| **Curve for Sample A.** | | | |
| **2-8C** | **2-8C** | **2-8C** | **2-8C** |
| **Day 0** | **Day 3** | **Day 6** | **Day 10** |

| **Conc. (ng/ml)** | **Conc. (ng/ml)** | **Conc. (ng/ml)** | **Conc. (ng/ml)** |
|---|---|---|---|
| 45.05 | 43.36 | 41.84 | 39.99 |
| | | | |
| **% Change** | **96.25** | **92.87** | **88.77** |
| | | | |
| **Sample B** | | | |
| **2-8C** | **37C** | **37C** | **37C** |
| **Day 0** | **Day 3** | **Day 6** | **Day 10** |

| **Conc. (ng/ml)** | **Conc. (ng/ml)** | **Conc. (ng/ml)** | **Conc. (ng/ml)** |
|---|---|---|---|
| 45.05 | 24.56 | 14.25 | 7.90 |
| | | | |
| **% Change** | **54.52** | **31.63** | **17.54** |
| | | | |
| | | | |

| **% Change based on Day 0 Calibration** | | | |
|---|---|---|---|
| **Curve for Sample C.** | | | |
| **2-8C** | **2-8C** | **2-8C** | **2-8C** |
| **Day 0** | **Day 3** | **Day 6** | **Day 10** |

| **Conc. (ng/ml)** | **Conc. (ng/ml)** | **Conc. (ng/ml)** | **Conc. (ng/ml)** |
|---|---|---|---|
| 43.55 | 43.12 | 38.08 | 37.65 |
| | | | |
| **% Change** | **99.01** | **87.44** | **86.45** |
| | | | |

| **Sample D** | | | |
|---|---|---|---|
| **2-8C** | **37C** | **37C** | **37C** |
| **Day 0** | **Day 3** | **Day 6** | **Day 10** |

| **Conc. (ng/ml)** | **Conc. (ng/ml)** | **Conc. (ng/ml)** | **Conc. (ng/ml)** |
|---|---|---|---|
| 43.55 | 40.86 | 35.85 | 34.44 |
| | | | |
| **% Change** | **93.82** | **82.32** | **79.08** |
| | | | |

The addition of 0.1% STANDAPOL® ES-1 to the matrix containing 0.1% porcine gelatin and adjustment of the pH from 4 to 6.8 has a significant effect on thermal stability of Troponin-I as observed after storing the samples at 37°C for ten days. Similar stabilities were observed for the samples stored at 2-8°C with or without STANDAPOL® ES-1.

### Example 2 - Comparison of STANDAPOL® ES-1 Lot-to-Lot Variability.

Three vendor lots of STANDAPOL® ES-1 were used in the preparation of analyte matrices. All matrices were based on a formulation: 0.025% porcine gelatin (Code 35745, Lot 74281P100, Abbott Laboratories, Abbott Park, IL), 0.175% of the selected STANDAPOL® ES-1, 2.5 mM NaPO₄ (as a buffer to maintain pH of the solution) and 0.1% ProClin 300 (primary active ingredient - methylchloroisothiazolone) at pH 7.0. ProClin 300 is an antimicrobial agent used to prohibit bacterial growth.

The three vendor lots of STANDAPOL® ES-1 were:
Lot 1- Code 88965, Lot 39267X102 (Abbott Laboratories)
Lot 2 - Code 88965, Lot 74497P100 (Abbott Laboratories)
Lot 3 - Code 88965, Lot 74498P101 (Abbott Laboratories)

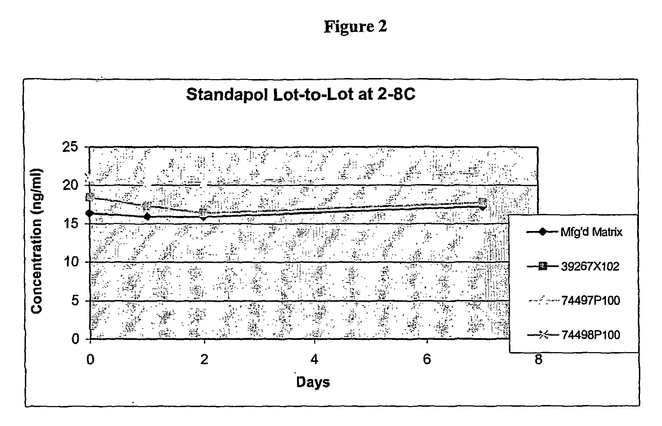

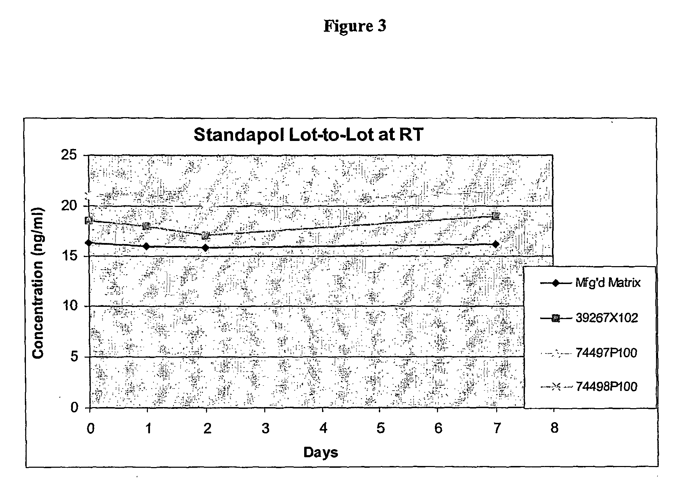

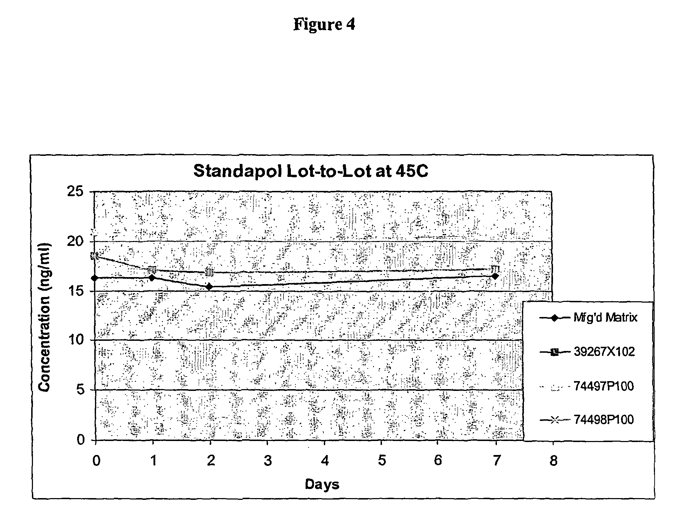

The three matrices (manufactured in Abbott Laboratories R&D facilities) were compared to a preparation manufactured in Bulk Solutions (Department 864, Abbott Laboratories). To this preparation was added Lot 1 to a concentration of 0.175%. Each of the matrices was used to dilute cardiac Troponin-I to a concentration between 15 ng/ml - 20 ng/ml. The samples were stored under three different temperature conditions: 2-8°C, room temperature and 45°C. The Troponin-I concentration of each standard was measured (via the AxSYM Troponin-I assay) on days 0, 1, 2 and 7. The percent Troponin-I activity remaining was determined. The results are tabulated in Tables 2-7 and depicted in Figures 2-4.

**Table 2. Troponin-I Stability at 2-8°C: STANDAPOL® ES-1 Lot-to-Lot Variability.**

| **STANDAPOL® ES-1 in Matrix** | **Concentrations at 2-8°C** | | | |
|---|---|---|---|---|
| | **Day 0** | **Day 1** | **Day 2** | **Day 7** |
| | (ng/ml) | (ng/ml) | (ng/ml) | (ng/ml) |
| **Bulk Soln Lot 1** | 16.34 | 15.94 | 15.91 | 17.23 |
| **Lot 1** | 18.48 | 17.30 | 16.36 | 17.75 |
| **Lot 2** | 21.01 | 20.41 | 20.31 | 21.75 |
| **Lot 3** | 21.00 | 20.85 | 21.44 | 21.06 |

**Table 3. Troponin-I Activity Remaining (%) following storage at 2-8°C.**

| **STANDAPOL® ES-1 in Matrix** | **% Activity Remaining at 2-8°C** | | | |
|---|---|---|---|---|
| | **Day 0** | **Day 1** | **Day 2** | **Day 7** |
| | (%) | (%) | (%) | (%) |
| **Bulk Soln Lot 1** | 100 | 98 | 97 | 105 |
| **Lot 1** | 100 | 94 | 89 | 96 |
| **Lot 2** | 100 | 97 | 97 | 104 |
| **Lot 3** | 100 | 99 | 102 | 100 |

After 7 days of storage at 2-8°C, the percent Troponin-I activity remaining varied with the lot of STANDAPOL® ES-1 used from 96% to 105%.

**Table 4. Troponin-I stability at room temperature: STANDAPOL® ES-1 Lot-to-Lot Variability.**

| **STANDAPOL® ES-1 in Matrix** | **Concentrations at Room Temperature** | | | |
|---|---|---|---|---|
| | **Day 0** | **Day 1** | **Day 2** | **Day 7** |
| | (ng/ml) | (ng/ml) | (ng/ml) | (ng/ml) |
| **Bulk Soln Lot 1** | 16.34 | 15.95 | 15.88 | 16.17 |
| **Lot 1** | 18.48 | 18.00 | 17.10 | 18.96 |
| **Lot 2** | 21.01 | 21.24 | 19.97 | 21.42 |
| **Lot 3** | 21.00 | 20.46 | 20.14 | 21.70 |

**Table 5. Troponin-I Activity Remaining (%) following storage at room temperature.**

| **STANDAPOL® ES1 in Matrix** | **% Activity Remaining at Room Temperature** | | | |
|---|---|---|---|---|
| | **Day 0** | **Day 1** | **Day 2** | **Day 7** |
| | (%) | (%) | (%) | (%) |
| **Bulk Soln Lot 1** | 100 | 98 | 97 | 99 |
| **Lot 1** | 100 | 97 | 93 | 103 |
| **Lot 2** | 100 | 101 | 95 | 102 |
| **Lot 3** | 100 | 97 | 96 | 103 |

After 7 days of storage at room temperature, the percent of Troponin-I activity remaining varied with the lot of STANDAPOL® ES1 used from 99% to 103%.

**Table 6. Troponin-I Stability at 45°C: STANDAPOL® ES-1 Lot-to-Lot Variability.**

| **STANDAPOL® ES-1 in Matrix** | **Concentrations at 45°C** | | | |
|---|---|---|---|---|
| | **Day 0** | **Day 1** | **Day 2** | **Day 7** |
| | (ng/ml) | (ng/ml) | (ng/ml) | (ng/ml) |
| **Bulk Soln Lot 1** | 16.34 | 16.31 | 15.45 | 16.57 |
| **Lot 1** | 18.48 | 17.06 | 16.80 | 17.23 |
| **Lot 2** | 21.01 | 21.14 | 20.06 | 20.69 |
| **Lot 3** | 21.00 | 21.04 | 20.08 | 20.83 |

**Table 7. Troponin-I Activity Remaining (%) following storage at 45°C.**

| **STANDAPOL® ES-1 in Matrix** | **% Activity Remaining at 45°C** | | | |
|---|---|---|---|---|
| | **Day 0** | **Day 1** | **Day 2** | **Day 7** |
| | (%) | (%) | (%) | (%) |
| **Manufactured w/1^{st} STANDAPOL® ES-1 added** | 100 | 100 | 95 | 101 |
| **1^{st} STANDAPOL® ES-1** | 100 | 92 | 91 | 93 |
| **2^{nd} STANDAPOL® ES-1** | 100 | 101 | 95 | 98 |
| **3^{rd} STANDAPOL® ES-1** | 100 | 100 | 96 | 99 |

After 7 days of storage at 45°C, the percent Troponin-I activity remaining varied with the lot of STANDAPOL® ES-1 used from 93% to 101%.

### Example 3 - Calibrator and Control Stability Study.

Calibrators are standard formulations containing known concentrations of Troponin-I and are used to define the calibration curve used in the assay. Controls are standard formulations also containing known concentrations of Troponin-I and were used to check the integrity of the calibration curve across the dynamic range of the assay.

Standard calibrators were prepared using the matrix formulation containing STANDAPOL® ES-1:
0.025% porcine gelatin (Code 35745, Lot 74281P103 (Sigma-Aldrich, Milwaukee, WI))
0.175% STANDAPOL® ES-1 (Code 88965, Lot 39267X102 (Cognis Corporation, Hoboken, N.J.))
2.5 mM NaPO₄
0.1% ProClin 300 (Suppleco) at pH 7.0, and
Troponin-I (Abbott Code 67911, Lot 78480P200, purchased from Scripps Laboratories, CA.) at the following Troponin-I concentrations: 0, 2.5, 5.0, 15, 30 and 50 ng/ml, designated A-F, respectively.

Similarly, three control standards were prepared at the following Troponin-I concentrations:
Low Control: 3.0 ng/ml
Medium Control: 10 ng/ml
High Control: 35 ng/ml

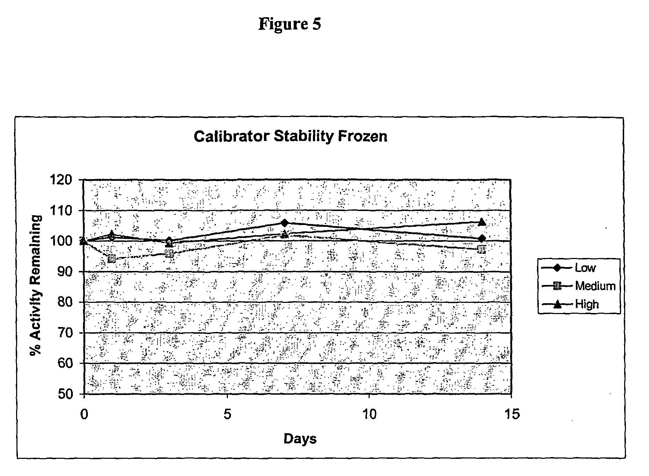

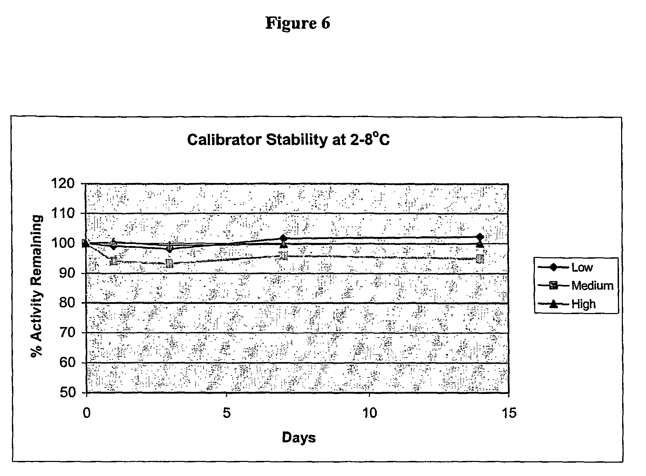

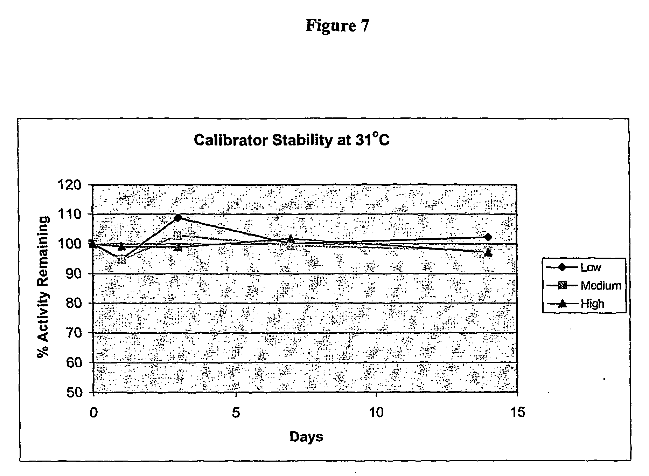

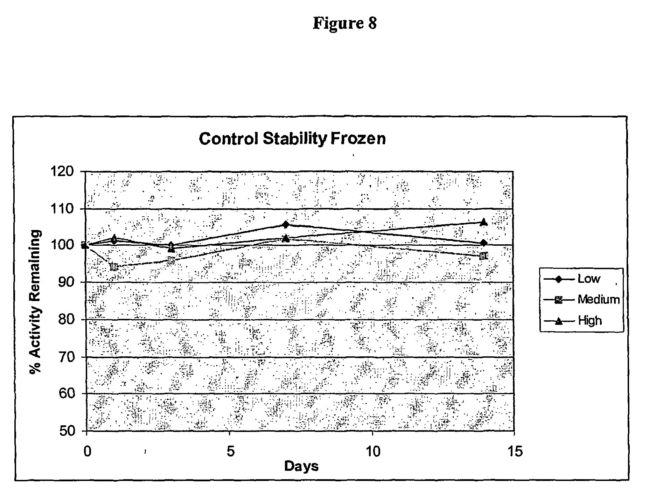

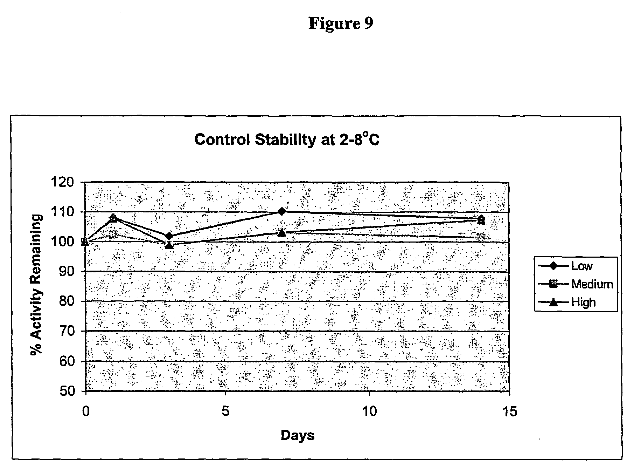

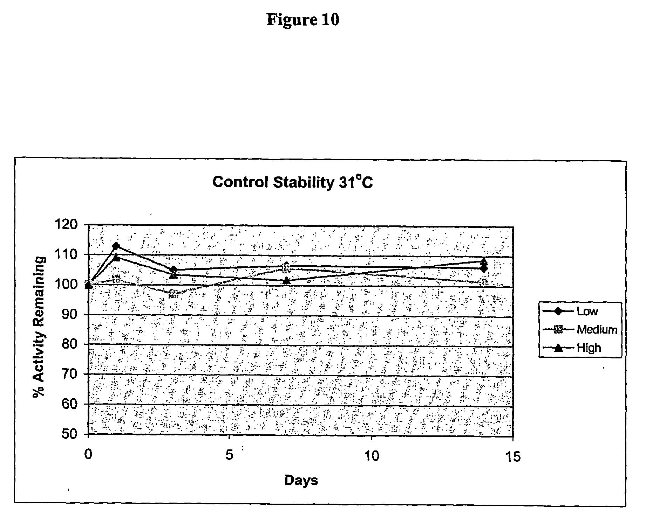

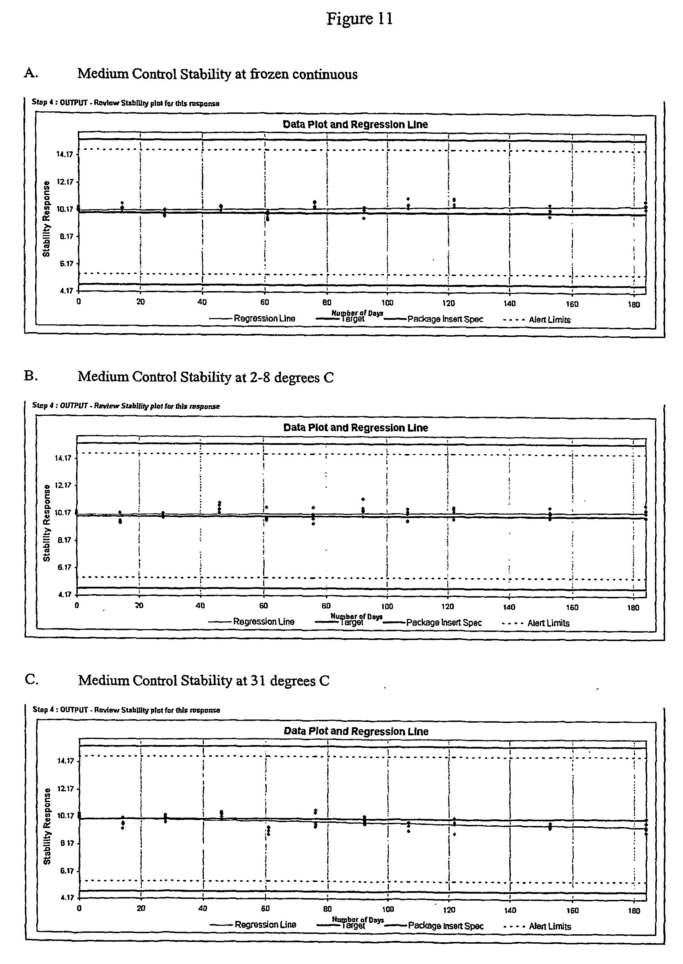

The complete set of standard calibrators and controls were each stored frozen, at 2-8°C and at 31°C. The temperature 31 °C was chosen as a worst case room temperature condition. Freshly thawed controls were used to evaluate the stability of the calibrators stored under various temperature conditions on days 0, 1, 3, 7 and 14. The results are tabulated in Tables 8-10 and depicted in Figures 5-7. Conversely, freshly thawed calibrators were used to evaluate the stability of controls that had been stored under the same temperature conditions on days 0, 1, 3, 7 and 14. The results are tabulated in Tables 11-13 and depicted in Figures 8-10. Additionally, the stability of the Medium Control at the various storage conditions was followed over 184 days as shown in Table 14 and Figure 11.

**Table 8. Calibrator Stability Stored Frozen. Troponin-I Control (%) Activity Remaining.**

| | **% Activity Remaining** | | | | |
|---|---|---|---|---|---|
| **Controls** | **Day 0** | **Day 1** | **Day 3** | **Day 7** | **Day 14** |
| **Low** | 100 | 101 | 100 | 106 | 101 |
| **Medium** | 100 | 94 | 96 | 102 | 97 |
| **High** | 100 | 102 | 99 | 102 | 106 |

**Table 9. Calibrator Stability Stored at 2-8°C. Troponin-I Activity Remaining.**

| | **% Activity Remaining using** | | | | |
|---|---|---|---|---|---|
| **Controls** | **Day 0** | **Day 1** | **Day 3** | **Day 7** | **Day 14** |
| **Low** | 100 | 99 | 98 | 102 | 102 |
| **Medium** | 100 | 94 | 93 | 96 | 95 |
| **High** | 100 | 100 | 99 | 100 | 100 |

**Table 10. Calibrator Stability Stored at 31°C. Troponin-I Activity Remaining.**

| | **% Activity Remaining** | | | | |
|---|---|---|---|---|---|
| **Controls** | **Day 0** | **Day 1** | **Day 3** | **Day 7** | **Day 14** |
| **Low** | 100 | 95 | 109 | 100 | 102 |
| **Medium** | 100 | 95 | 103 | 100 | 98 |
| **High** | 100 | 99 | 99 | 102 | 97 |

**Table 11. Control Stability: Troponin-I Activity Remaining for Low, Medium and High Controls Stored Frozen.**

| | **% Activity Remaining using frozen cals** | | | | |
|---|---|---|---|---|---|
| **Controls** | **Day 0** | **Day 1** | **Day 3** | **Day 7** | **Day 14** |
| **Low** | 100 | 101 | 100 | 106 | 101 |
| **Medium** | 100 | 94 | 96 | 102 | 97 |
| **High** | 100 | 102 | 99 | 102 | 106 |

**Table 12. Control Stability: Troponin-I (%) Activity Remaining for Low, Medium and High Controls Stored at 2-8°C.**

| | **% Activity Remaining using frozen stored cals** | | | | |
|---|---|---|---|---|---|
| **Controls** | **Day 0** | **Day 1** | **Day 3** | **Day 7** | **Day 14** |
| **Low** | 100 | 108 | 102 | 110 | 108 |
| **Medium** | 100 | 102 | 99 | 103 | 101 |
| **High** | 100 | 108 | 99 | 103 | 107 |

**Table 13. Control Stability: Troponin-I Activity Remaining for Low, Medium and High Controls Stored at 31°C.**

| | **% Activity Remaining using frozen stored cals** | | | | |
|---|---|---|---|---|---|
| **Controls** | **Day 0** | **Day 1** | **Day 3** | **Day 7** | **Day 14** |
| **Low** | 100 | 113 | 105 | 107 | 106 |
| **Medium** | 100 | 102 | 97 | 106 | 101 |
| **High** | 100 | 109 | 103 | 102 | 109 |

**Table 14. Control Stability: Troponin-I Activity Remaining for Medium Control Stored at Frozen, 2-8°C and 31°C.**

| | **% Activity Remaining using frozen stored cals** | | | | |
|---|---|---|---|---|---|
| **Storage** | **Day 0** | **Day 14** | **Day 28** | **Day 92** | **Day 184** |
| **Frozen** | 100 | 98 | 97 | 94 | 97 |
| **2-8°C** | 100 | 97 | 98 | 103 | 102 |
| **31°C** | 100 | 96 | 97 | 97 | 93 |

Calibrator stability, stored under various temperature conditions, was assessed over 14 days using freshly thawed controls. Troponin-I activity remaining after 14 days ranged between 97-106%. Calibrators stored at 2-8°C and 31°C demonstrated similar Troponin-I % activity remaining ranging from 95-102% and 97-102%, respectively after 14 days.

Control stability stored under frozen, 2-8°C and 31°C conditions showed comparable retention of activity. Frozen controls retained between 97-106% activity after 14 days. Controls stored at 2-8°C and 31°C yielded Troponin-I activities from 101-109% after 14 days as compared to activity measured on day 0. The medium control stored under frozen, 2-8°C and 31°C conditions retained 97,102 and 93% activity, respectively after 184 days.

The sodium polyoxyethylene (1) lauryl sulfate was found not to cause any carryover effects from sample to sample in the AxSYM instrument system.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

While some potential advantages and objects have been expressly identified herein, it should be understood that some embodiments of the invention may not provide all, or any, of the expressly identified advantages and objects.

Preferred embodiments of this invention are described herein, including the best mode known to the inventors for carrying out the invention. Of course, variations of those preferred embodiments will become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A diagnostic assay standard comprising:
an aqueous solution of Troponin-I protein; and
a matrix comprising at least one anionic surfactant, wherein the anionic surfactant is sodium polyoxyethylene (1) lauryl sulfate.

2. A diagnostic assay standard according to claim 1 comprising:
an aqueous solution containing a complex of Troponin-I and Troponin-C protein; and
a matrix comprising at least one anionic surfactant, wherein the anionic surfactant is sodium polyoxyethylene (1) lauryl sulfate.

3. The diagnostic assay standard of any one of claims 1 and 2 wherein the standard is stable at room temperature for at least six months.

4. The diagnostic assay standard of any one of claims 1 and 2 wherein the matrix further comprises porcine gelatin.

5. The diagnostic assay standard of any one of claims 1 and 2 wherein the matrix further comprises a compound selected from the group consisting of sodium chloride and sodium phosphate.

6. The diagnostic assay standard of any one of claims 1 and 2 wherein the matrix further comprises a pH buffer added to hold the pH within a range of 6.8 to 7.2.

7. The diagnostic assay standard of any one of claims 1 and 2 wherein the sodium polyoxyethylene (1) lauryl sulfate is in a concentration of 0.1% to 0.25%.

8. A method for stabilizing Troponin in an aqueous solution, the method comprising the steps of:
preparing an aqueous solution of at least one anionic surfactant, wherein the anionic surfactant is sodium polyoxyethylene (1) lauryl sulfate;
adding Troponin-I protein; and
storing the solution at room temperature.

9. A method for stabilizing Troponin in an aqueous solution according to claim 8, the method comprising the steps of:
preparing an aqueous solution of at least one anionic surfactant, wherein the anionic surfactant is sodium polyoxyethylene (1) lauryl sulfate;
adding a Troponin-I protein and Troponin-C protein, either individually or together as a single complex; and
storing the solution at room temperature.

## Patentansprüche

1. Ein Diagnosetest-Standard, der Folgendes umfasst:
eine wässerige Lösung von Troponin-I-Protein, und
eine Matrix, die mindestens einen anionischen grenzflächenaktiven Stoff umfasst, worin der anionische grenzflächenaktive Stoff Natriumpolyoxyethylen(1)-laurylsulfat ist.

2. Ein Diagnosetest-Standard gemäß Anspruch 1, der Folgendes umfasst:
eine wässerige Lösung, die einen Komplex aus Troponin-I- und Troponin-C-Protein enthält, und
eine Matrix, die mindestens einen anionischen grenzflächenaktiven Stoff umfasst, worin der anionische grenzflächenaktive Stoff Natriumpolyoxyethylen(1)-laurylsulfat ist.

3. Der Diagnosetest-Standard gemäß einem beliebigen der Ansprüche 1 und 2, worin der Standard bei Raumtemperatur über mindestens sechs Monate stabil ist.

4. Der Diagnosetest-Standard gemäß einem beliebigen der Ansprüche 1 und 2, worin die Matrix weiter Schweinegelatine umfasst.

5. Der Diagnosetest-Standard gemäß einem beliebigen der Ansprüche 1 und 2, worin die Matrix weiter eine Verbindung umfasst, die gewählt ist aus der Gruppe bestehend aus Natriumchlorid und Natriumphosphat.

6. Der Diagnosetest-Standard gemäß einem beliebigen der Ansprüche 1 und 2, worin die, Matrix weiter einen pH-Puffer umfasst, der hinzugefügt wird, um den pH innerhalb eines Bereichs von 6,8 bis 7,2 zu halten.

7. Der Diagnosetest-Standard gemäß einem beliebigen der Ansprüche 1 und 2, worin das Natriumpolyoxyethylen(1)-laurylsulfat in einer Konzentration von 0,1% bis 0,25% vorliegt.

8. Ein Verfahren zur Stabilisierung von Troponin in einer wässerigen Lösung, wobei das Verfahren folgende Schritte umfasst:
Herstellung einer wässerigen Lösung von mindestens einem anionischen grenzflächenaktiven Stoff, worin der anionische grenzflächenaktive Stoff Natriumpolyoxyethylen(1)-laurylsulfat ist,
Hinzufügen von Troponin-I-Protein, und
Lagerung der Lösung bei Raumtemperatur.

9. Ein Verfahren zur Stabilisierung von Troponin in einer wässerigen Lösung gemäß Anspruch 8, wobei das Verfahren folgende Schritte umfasst:
Herstellung einer wässerigen Lösung von mindestens einem anionischen grenzflächenaktiven Stoff, worin der anionische grenzflächenaktive Stoff Natriumpolyoxyethylen(1)-laurylsulfat ist,
Hinzufügen eines Troponin-I-Proteins und Troponin-C-Proteins, entweder einzeln oder gemeinsam als ein einziger Komplex, und
Lagerung der Lösung bei Raumtemperatur.

## Revendications

1. Etalon de dosage diagnostique comprenant :
une solution aqueuse de protéine troponine-I ; et
une matrice comprenant au moins un tensioactif anionique, où le tensioactif anionique est le laurylsulfate de polyoxyéthylène (1) sodique.

2. Etalon de dosage diagnostique selon la revendication 1 comprenant :
une solution aqueuse contenant un complexe de protéines - troponine-I et troponine-C ; et
une matrice comprenant au moins un tensioactif anionique, où le tensioactif anionique est le laurylsulfate de polyoxyéthylène (1) sodique.

3. Etalon de dosage diagnostique selon l'une quelconque des revendications 1 et 2 où l'étalon est stable à température ambiante pendant au moins six mois.

4. Etalon de dosage diagnostique selon l'une quelconque des revendications 1 et 2 où la matrice comprend en outre de la gélatine de porc.

5. Etalon de dosage diagnostique selon l'une quelconque des revendications 1 et 2 où la matrice comprend en outre un composé sélectionné dans le groupe constitué du chlorure de sodium et du phosphate de sodium.

6. Etalon de dosage diagnostique selon l'une quelconque des revendications 1 et 2 où la matrice comprend en outre un tampon de pH ajouté pour maintenir le pH dans une fourchette de 6,8 à 7,2.

7. Etalon de dosage diagnostique selon l'une quelconque des revendications 1 et 2 où le laurylsulfate de polyoxyéthylène (1) sodique est à une concentration de 0,1 % à 0,25 %.

8. Procédé de stabilisation de troponine en solution aqueuse, le procédé comprenant les étapes de :
préparation d'une solution aqueuse d'au moins un tensioactif anionique, où le tensioactif anionique est le laurylsulfate de polyoxyéthylène (1) sodique ;
addition de la protéine de troponine-I ; et
stockage de la solution à température ambiante.

9. Procédé de stabilisation de troponine en solution aqueuse selon la revendication 8, le procédé comprenant les étapes de :
préparation d'une solution aqueuse d'au moins un tensioactif anonique, où le tensioactif anonique est le laurylsulfate de polyoxyéthylène (1) sodique ;
addition d'une protéine de troponine-I et d'une protéine de troponine-C, soit individuellement, soit ensemble sous forme d'un seul complexe ; et
stockage de la solution à température ambiante.
